# EUROPEAN PATENT SPECIFICATION

(11) **EP 2 081 906 B1**
(45) Date of publication and mention of the grant of the patent: **22.09.2010**
(21) Application number: 07821013.5
(22) Date of filing: 08.10.2007
(51) Int. Cl.: C07D 215/40, A61K 31/47, A61P 33/02

(54) **SITAMAQUINE TOSYLATE FOR THE TREATMENT OF LEISHMANIASIS**
SITAMAQUINTOSYLAT ZUR BEHANDLUNG VON LEISHMANIASE
TOSYLATE DE SITAMAQUINE POUR LE TRAITEMENT DE LA LEISHMANIOSE

(30) Priority: 10.10.2006 GB 0620005
(43) Date of publication of application: 29.07.2009
(73) Proprietor: Glaxo Group Limited, Greenford Middlesex UB6 0NN (GB)
(72) Inventor: SEARLE, Andrew, David, Stevenage Hertfordshire SG1 2NY (GB)
(74) Representative: Giddings, Peter John
(86) International application number: PCT/EP2007/060640
(87) International publication number: WO 2008/043726

(56) References cited:
- US-A- 4 209 519
- US-A- 4 659 708
- JHA ET AL: "A Phase II Dose-Ranging Study of Sitamaquine for the Treatment of Visceral Leishmaniasis in India" AM. J. TROP. MED. HYG., vol. 73, no. 6, 2005, pages 1005-1011, XP002462669

## Description

The present invention relates to the salt of an 8-amino-6-methoxy quinoline compound and its use in therapy for the treatment of leishmaniasis.

Leishmaniasis is caused by intracellular species and subspecies of the protozoal genus *Leishmania.* It is most commonly transmitted to man by the bites of infected female sandflies. Of the spectrum of diseases associated with the various species of *Leishmania,* visceral leishmaniasis, also known as kala-azar, is the most life threatening and leads to infection of the liver, spleen and bone marrow. The parasite amastigotes survive and grow in host macrophages, the very cells that should be destroying them. Leishmaniasis is endemic in tropical and sub-tropical countries, affecting more than 12 million people, and in the absence of treatment there is high (>90%) mortality. A number of treatments have been made available for the treatment of leishmaniasis. For example, sodium stibogluconate; amphoterecin B (alone or in lipid formulations), and aminosidine (paromomycin) - but all these treatments however are associated with high costs and/or serious side effects.

8-amino-6-methoxy lepidine compounds are also known in the art as potentially useful in the treatment of leishmania (see US Patent 4209519) and one compound falling within this class, sitamaquine has been shown to have such potential in clinical trials (Current Optimism in Investigational Drugs, 2002 3(10) 1446-1452).

Sitamaquine is the compound N,N-diethyl-N'-(6-methoxy-4-methyl-8-quinolinyl),6-hexane diamine, of structure (I).

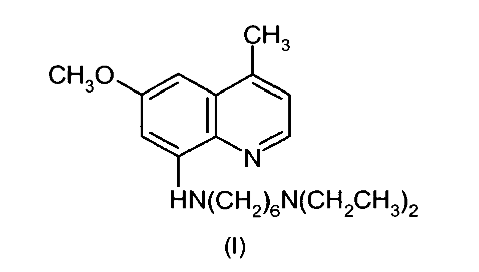

Early clinical trials of this compound have been conducted using the dihydrochloride salt form of the compound, but it has been found that this salt has poor developability characteristics, that is to say it is hygroscopic at about 60% relative humidity, and has a tendency to give mixed polymorphic forms during its production. This means that reproducibility of standard batches of drug is very difficult, and that the drug would have a very short shelf life in those countries where it is particularly needed.

There is therefore a need for a form of sitamaquine that has acceptable developability characteristics, and that will allow for the reliable production of consistent and appropriately stable formulations to allow treatment of this serious disease.

The present invention thus provides in a first aspect, sitamaquine in the form of the tosylate salt of structure I(A)

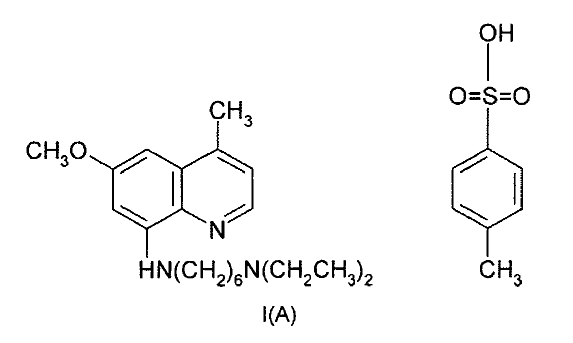

The compound of structure I(A) may also be referred to herein as sitamaquine tosylate.

It will be appreciated that sitamaquine tosylate may exist in more than one polymorphic forms, and it is intended that all such forms are included within the scope of this invention.

Sitamaquine tosylate can be prepared according to the process described herein starting from 2-methoxy-5-chloro-6-methoxy 8-nitro lepidine (which itself can be prepared according to the processes described in WO97/13753 ).

In a further aspect, there is provided a process for the preparation of sitamaquine tosylate that comprises reaction of a compound of formula (II) 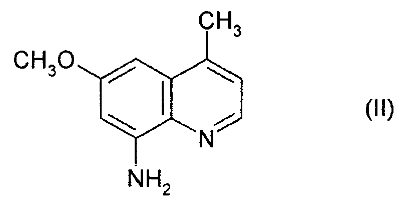 with a compound of formula (III)

Br(CH₂)₆N(CH₂CH₃)₂.HBr. (III)

to form sitamaquine free base, and then thereafter forming the tosylate salt by reaction with p-toluene sulphonic acid monohydrate. The details of this process are described herein.

It will be appreciated that variations in solvents, reagents and reaction candidates can also be used to produce sitamaquine tosylate, and these variations are also contemplated with the scope for the present invention.

Sitamaquine tosylate is expected to be useful in medicine, in particular in the treatment of leishmaniasis in humans. In a further aspect there is therefore provided the use of sitamaquine tosylate in medicine. In a further aspect there is provided a method of treatment of leishmaniasis in humans, that comprises administering to a subject in need therefore an effective amount of sitamaquine tosylate. When used for the treatment of leishmaniasis, sitamaquine tosylate can be administered alone or in combination with another active compound having anti-leishmanial activity. Such compounds will be apparent to those skilled in the art and include for example, amphotericin B, paromomycin, and miltefosine.

When used in medicine sitamaquine tosylate may be formulated into a pharmaceutical composition for administration in any convenient and pharmaceutically acceptable way. In a further aspect there is therefore provided a pharmaceutical composition comprising sitamaquine tosylate and a pharmaceutically acceptable carrier.

The pharmaceutical compositions of the invention include those in a form adapted for oral, topical or parenteral use.

The compositions may be in the form of tablets, capsules, powders, granules, lozenges, and liquid preparations, such as oral or sterile parenteral solutions or suspensions.

Tablets and capsules for oral administration may be in unit dose presentation form, and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol, mannitol or glycine; tabletting lubricants, for example magnesium stearate, talc, polyethylene glycol or silica; disintegrants, for example potato starch; wetting agents such as sodium lauryl sulphate; compression aids such as microcrystalline cellulose, and flow aids such as silicon dioxide. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and, if desired, conventional flavouring or colouring agents.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing.

Typically, compositions are administered in dosage units. Each dosage unit may contain from 10-150 mg of the active ingredient. The dosage as employed for adult human treatment is expected to range from 1 to 2 mg/kg per day. Suitably the dosage is expected to be from 1.5 to 2 mg/kg per day. For a subject of 70kg weight, this equates to a daily dose of from 70 - 140mg per day. The period of treatment is expected to vary depending on the severity of the condition, but is routinely anticipated to be a period of between 14 and 21 days.

The following examples serve to illustrate the invention.

### Preparation 1: 6-(diethylamino)-1-hexanol,

6-Chlorohexanol (20kg) and diethylamine (26.8kg) are added to dimethyl sulphoxide (40L) with stirring. The reaction mixture is heated to reflux temperature of 60°C and maintained for 8¹/₂ hr at reflux at 60-115°C (Reflux temperature increases as reaction progresses). After completion of reaction, cool the reaction mixture to 60-70°C and distil off excess diethyl amine under vacuum.

To the cooled reaction mixture of 50-60°C, water (60L) is added and the resulting reaction mixture is further cooled to 25-35C. 50% aqueous sodium hydroxide (10L) is added to the reaction mixture at 25-35°C over 10-15 min and then cooled to 20-25°C. To the mixture, pet ether (20L) is added at 20-25°C and stirred for about 10 min. The resulting organic layer is separated and washed with water (2 x 12L), each time stirring for about 2 min. The organic solution is concentrated under vacuum to remove pet ether at 65-70°C. The product is cooled to 25-30°C to give the title compound (21.4-24.2kg).

400MHz NMR in CDCl₃. TMS as reference at 0.0 ppm.
δ(ppm): 1.02 (6 H, t, *J*=7.1 Hz), 1.26 - 1.41 (4 H, m), 1.42 - 1.51 (2 H, m), 1.53 - 1.62 (2 H, m), 2.37 - 2.43 (2 H, m), 2.52 (4 H, q, *J*=7.1 Hz), 3.63 (2 H, t, J=6.5 Hz)

### Preparation 2: (6-bromohexyl)diethylamine hydrobromide

Aqueous hydrobromic acid (43kg; @ 62%) is added uniformly to 6-(diethylamino)-1-hexanol (20kg) with stirring over 30-60 minutes controlling the resulting exotherm below 60°C. The reaction mixture is then heated to 100°C and maintained for 2hr at 98-102°C. The reaction mixture is cooled to 75-80°C and then toluene (160L) is charged.

The reaction is heated to reflux and water is removed azeotropically at reflux temperature of 90-110°C and then 60% of input toluene is distilled off at 100-110°C. The reaction mixture is cooled to 80°C and then the remaining toluene is distilled off under vacuum.

Ethyl acetate (120L) is added to the hot reaction mixture and temperature is adjusted to 50-60°C. To the ethyl acetate solution, anhydrous sodium sulphate (1.2kg) is added and stirred for 3-5 min, followed by sodium bicarbonate (2.4kg) and stirred for 3-5 min. To the total mixture, activated charcoal (2kg) is added at 50-60°C. The mixture is heated to 65-70°C and stirred for 50-60 min. The hot reaction mixture is filtered through a celite bed and washed with hot ethyl acetate (8L) at 65-70°C. The total filtrate is cooled to 0-5°C under a nitrogen atmosphere and stirred for 50-60 min. The product is filtered, and washed with cold ethyl acetate (12L) at 0-5°C. The product is dried at 40-45°C under vacuum to give the title compound (25.6-30.4kg).

400MHz NMR in DMSO-d6. TMS as reference at 0.0 ppm.
δ(ppm): 1.21 (6H, t, *J*=7.2 Hz), 1.28 - 1.38 (2 H, m), 1.38 - 1.47 (2 H, m), 1.60 - 1.69 (2 H, m), 1.77 - 1.86 (2H, m), 2.98 - 3.06 (2 H, m), 3.12 (4H, dt, *J*=12.0, 7.1 Hz), 3.55 (2 H, t, *J*=6.6 Hz), 9.40 (1H, s)

### Example 1: 5-chloro-2,6-dimethoxy-4-methyl-8-nitroquinolinone

5-chloro-4,8-dimethyl-2,6-bis(methyloxy)quinoline (10kg) (which can be prepared according to the procedures described in W/O 97/13753) is charged to acetic acid (30L) at 25-30°C. To the mixture, hydrobromic acid (13.2kg; @62%) is added under stirring at 25-35°C over 10 to 15 min. The mixture is heated to 75-80°C and then maintained at 75-85 °C for 1 hr. After completion of reaction, the hot reaction mixture at 75-85 °C is added to water (30L) at 15-20°C maintaining the temperature below 40°C. The mixture is maintained for 30 min at 23-30°C. The product is filtered, washed with water (30L) to neutral pH followed by methanol (20L) and dried at 50-55°C to give the title compound (8.5-9.5kg).

500MHz NMR in DMSO-d6. DMSO-d5 as reference at 2.50 ppm.
δ(ppm): 2.81 (3 H, s), 3.98 (3 H, s), 6.72 (1 H, s), 8.14 (1H, br.s), 11.18 (1 H, br.s)

### Example 2: 2,5-Dichloro-6-methoxy-8-nitro-4-methyl-quinoline

5-chloro-2,6-dimethoxy-4-methyl-8-nitroquinolinone (8.5kg) is added to phosphorus oxychloride (32.5kg) at 55-60°C over a period of 30-45 min under stirring. The mixture is heated gradually to 60-65°C then heating is stopped and the mixture is allowed to warm to 95°C without heating or otherwise gradually heated to reach 90-95°C. The reaction mixture is further heated to reflux temperature (105-110°C) and maintained for 2hr 30 min. The hot reaction mixture is quenched into cold water of 15-20°C (150L) below 70°C. The mixture is cooled to 20-25°C, stirred for 30-45 min at 20-25°C, filtered and the product is washed with water (35L) to neutral pH and dried at 50-55°C to give the title compound (8.0-9.0kg)

500MHz NMR in DMSO-d6. DMSO-d5 as reference at 2.50 ppm.
δ(ppm): 3.00 (3 H, s), 4.05 (3 H, s), 7.66 (1 H, s), 8.46 (1 H, s)

### Example 3: 4-methyl-6-(methyloxy)-8-quinolinamine

A mixture of anhydrous sodium acetate (9.1kg) and 2,5-Dichloro-6-methoxy-8-nitro-4-methyl-quinoline (12.5kg) in ethyl acetate (62.5L) are added to a hydrogenator. The equipment is evacuated with about 2kg/cm² nitrogen three times, under stirring. To the mixture, a slurry of 5% Pd/C (2.3kg 50% wet) in ethyl acetate (12.5L) is added into the hydrogenator under nitrogen atmosphere. The equipment is evacuated with about 2 kg/cm² nitrogen three times followed by with hydrogen of 2 kg/cm² three times with stirring. After evacuation, hydrogen with 1-6 kg/cm² pressure is fed into the reaction mixture at about 20°C. The exothermic temperature of reaction mixture is controlled below 80°C. When the exotherm has ceased, the reaction mixture is heated to 80-90°C with -9 kg/cm² of hydrogen pressure. The reaction mixture is heated to 90-95°C and maintained with 9-10 kg/cm² pressure for 3 hr.

The reaction mixture is cooled to 40-45°C and hydrogen is evacuated with nitrogen of about 2 kg/cm² pressure three times. The reaction mixture is filtered under a nitrogen atmosphere and washed with ethyl acetate (25L) The total filtrate is treated with dil. sulphuric acid (made from water (112.5L) and conc. sulphuric acid (6.5kg) for 20-25 min at 25-35°C then the aqueous layer is separated. The organic layer is washed with water (12.5L) and then the combined aqueous solution is charged into the reactor and heated under vacuum of about 600 mm/hg for 1 hr 30 min at 35-40 °C to remove residual ethyl acetate in aqueous solution.

The aqueous solution is treated with sodium hydroxide (19L) at 25-35°C to adjust pH to >9. The mixture is cooled to 20-25°C, stirred for 15 min and filtered. The product is washed with water to neutral pH and dried at 45-50°C to give the title compound (6.2-7.4kg).

500MHz NMR in DMSO-d6. DMSO-d5 as reference at 2.50 ppm.
δ(ppm): 2.54 (3 H, s), 3.82 (3 H, s), 5.94 (2 H, s), 6.49 (2 H, dd, *J*=11.9, 2.1 Hz), 7.25 (1 H, d, *J*=4.0 Hz), 8.40 (1 H, d, *J*=4.3 Hz)

### Example 4: N,N-diethyl-N'-[4-methyl-6-(methyloxy)-8-quinolinyl]-1,6-hexanediamine tosylate (Sitamaquine tosylate)

4-methyl-6-(methyloxy)-8-quinolinamine (1kg) is charged into a reactor, which contains toluene (4L), followed by activated carbon (0.08kg). The reaction mixture is azeotropically dried by distilling 1/4^{th} volume of toluene at 105 to 115°C. The reaction mixture is filtered at 70 to 80°C, followed by toluene wash (0.5L). Toluene (9L) is charged into another reactor, followed by (6-bromohexyl)diethylamine hydrobromide, (2.12kg). The temperature is raised to 105 to 115°C and a portion of (~1/4^{th} volume) of the solvent is distilled off. Then it is cooled to 101 to 105°C and tri n-butylamine (0.065kg) is charged. The solution of 6-methoxy-4-methyl-8-quinolinamine is added at 101 to 103°C and the temperature is raised to 105 to 115°C. After 5 hr stirring under azeotropic reflux, the mixture is cooled to 81 to 85°C and purified water (4L) is charged. The mixture is further cooled to 65 to 75°C and sodium hydroxide solution (1.2L) is added at 65 to 75°C over 15 to 25 min. The bottom layer is separated and discarded. The toluene layer is washed three times with sodium chloride solution (made from 0.25kg in 2.5L of water) at 65 to 75°C. Purified water (10L) is added to the organic layer and the pH of the reaction mass is adjusted to 4.00 to 4.50 with orthophosphoric acid (0.52L) at 22 to 28°C. The bottom aqueous layer is collected and the toluene layer is discarded. The aqueous layer is washed with ethyl acetate (6 x 2.5L). The pH of the aqueous layer is adjusted to 10.00 to 10.50 with liquor ammonia (4L) at 23 to 28°C. The product is extracted into toluene (3 x 1.5L). The toluene is dried with sodium sulphate (0.6kg) and decolorized with activated carbon (0.2kg then filtered, washing with toluene (0.5L). The mass is concentrated by distilling toluene at below 50°C under vacuum to give crude free base form of the title compound (1.6kg).

Isopropyl alcohol (6.5L) and crude free base (1.6kg) are charged into a reactor, followed by p-toluenesulphonic acid monohydrate (0.92kg). The temperature is raised to 43 to 47°C to get clear solution and stirred for 4 hr at 43 to 47°C. The mass is cooled to 25 to 35°C and seeded with pure Sitamaquine tosylate (0.003kg). The mixture is cooled to 1 to 5°C and stirred for 1 hr at 1 to 5°C and filtered, followed by isopropyl alcohol washing (1 L). The wet product is charged into a reactor with Isopropyl alcohol (1.5L). The reaction mass temperature is raised to 43 to 47°C and stirred for 15 min. to get clear solution. The mass is gradually cooled to 1 to 5°C, stirred for 1 hr at 1 to 5°C and filtered, followed by chilled IPA washing (1.5L). The wet product is dried at below 40°C under vacuum to give the title compound (1.21 kg).

The product can be purified as follows. Isopropyl alcohol (12L) and crude title compound (1.21kg) are charged into a glass flask. The temperature is raised to 43 to 47°C and stirred for 15 min. to get clear solution. The mass is decolorized with activated carbon (0.036kg) at 43 to 47°C then filtered and washed with isopropyl alcohol (0.2L). The mass is cooled to 1-5°C, stirred for 1 hr at 1 to 5°C and then filtered and washed with isopropyl alcohol (0.4L) then dried at below 40° to give the title compound (1.15kg).

400MHz NMR in CDCl₃. TMS as reference at 0.0 ppm.
δ(ppm): 1.30 (6 H, t, *J*=7.3 Hz), 1.34 - 1.41 (2 H, m), 1.41 - 1.50 (2 H, m), 1.65 - 1.75 (4 H, m), 2.31 (3 H, s), 2.58 (3 H, s), 2.90 - 3.00 (2 H, m), 3.10 (4 H, q, *J*=7.3 Hz), 3.19 - 3.28 (2 H, m), 3.91 (3 H, s), 6.18 (1 H, br.s), 6.27 (1 H, d, *J*=2.4 Hz), 6.42 (1 H, d, *J*=2.4 Hz), 7.13 - 7.18 (3 H, m), 7.76 (2 H, d, *J*=8.1 Hz), 8.40 (1 H, d, *J*=4.4 Hz), 10.28 (1H, br.s)

### Example 5: Pharmaceutical Composition

A 10 mg tablet composition comprising sitamaquine tosylate for oral use is prepared from the following ingredients:

| **Component** | **Quantity mg** | **Function** | **Reference to Standard** |
|---|---|---|---|
| Sitamaquine tosylate | 15.0 | Active | |
| Mannitol | 88.5 | Filler | Ph.Eur. or USP |
| Microcrystalline Cellulose | 45.0 | Compression Aid | Ph.Eur. or USNF |
| Magnesium Stearate | 1.5 | Lubricant | Ph.Eur. or USNF |
| Opadry White OY-S-288763,4 | 4.5 | Film Coat | Supplier |
| **Total** | **154. 5** | **-** | **-** |

## Claims

1. Sitamaquine tosylate of structure (1A):

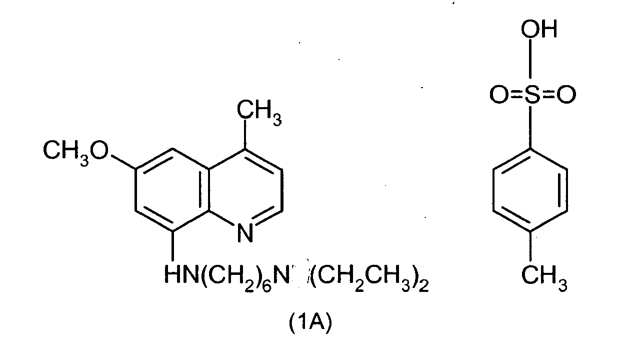

2. A pharmaceutical composition comprising the compound according to claim 1 and a pharmaceutically acceptable carrier.

3. A compound according to claim 1 for use in therapy.

4. A compound according to claim 1 for use in the treatment of leishmaniasis.

## Patentansprüche

1. Sitamaquintosylat der Formel (1A):

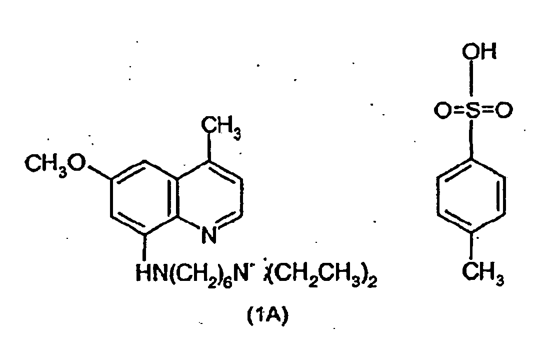

2. Pharmazeutische Zusammensetzung, die aus der Verbindung nach Anspruch 1 und einem pharmazeutisch verträglichen Träger besteht.

3. Verbindung nach Anspruch 1 zur Verwendung in der Therapie.

4. Verbindung nach Anspruch 1 zur Verwendung bei der Behandlung von Leishmaniosen.

## Revendications

1. Tosylate de sitamaquine de structure (1A):

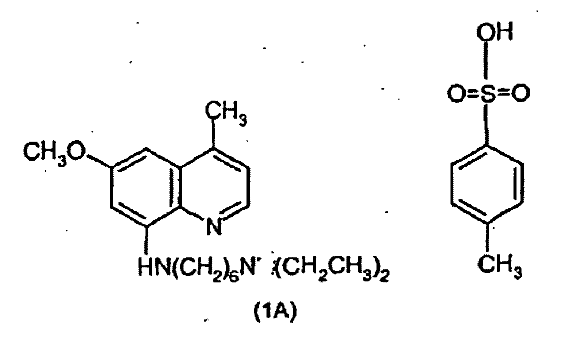

2. Une composition pharmaceutique comprenant le composé selon la revendication 1 et un véhicule acceptable au plan pharmaceutique.

3. Un composé selon la revendication 1 pour application en thérapeutique.

4. Un composé selon la revendication 1 pour application dans le traitement de la leishmaniose.
